# EUROPÄISCHE PATENTSCHRIFT

(11) **EP 4 245 588 B1**
(45) Veröffentlichungstag und Bekanntmachung des Hinweises auf die Patenterteilung: **09.07.2025**
(21) Anmeldenummer: 23153499.1
(22) Anmeldetag: 26.01.2023
(51) Int. Cl.: B60K 35/10, B60K 35/22, G06F 3/01, A61B 5/18, B60K 35/28, B60K 35/60

(54) **FAHRERKABINE FÜR EINE LANDWIRTSCHAFTLICHE ARBEITSMASCHINE**
DRIVER'S CAB FOR AN AGRICULTURAL MACHINE
CABINE DE CONDUCTEUR POUR MACHINE DE TRAVAIL AGRICOLE

(30) Priorität: 10.03.2022 DE 102022105592
(43) Veröffentlichungstag der Anmeldung: 20.09.2023
(73) Patentinhaber: CLAAS Selbstfahrende Erntemaschinen GmbH, 33428 Harsewinkel (DE)
(72) Erfinder: Frenser, Reinhard, 33378 Rheda-Wiedenbrück (DE); Jeppe, Eckehardt, 34289 Zierenberg (DE)
(74) Vertreter: CLAAS Gruppe

(56) Entgegenhaltungen:
- WO-A1-2021/222384
- DE-A1- 102020 120 201
- US-A1- 2014 276 090
- US-A1- 2021 056 306
- US-A1- 2021 190 515

## Beschreibung

Die vorliegende Anmeldung betrifft eine Fahrerkabine für eine landwirtschaftliche Arbeitsmaschine gemäß dem Oberbegriff von Anspruch 1. Ferner betrifft die vorliegende Anmeldung eine landwirtschaftliche Arbeitsmaschine gemäß dem Oberbegriff von Anspruch 17 sowie ein Verfahren zu deren Betrieb gemäß dem Oberbegriff von Anspruch 18.

Die Fahrerkabine umfasst eine Frontscheibe, die sich zwischen zwei Tragsäulen erstreckt. Letztere gehören zu einer Tragstruktur, die typischerweise Tragsäulen in Form von A-Säulen und B-Säulen, sich zwischen den Tragsäulen erstreckende Raumabschlusselemente, typischerweise in Form von Glasscheiben, sowie ein Dach umfasst. Insbesondere kann sich die Frontscheibe zwischen zwei A-Säulen erstrecken und einen vorderen räumlichen Abschluss der Fahrerkabine bilden, während sich Seitenscheiben auf beiden Seiten der Fahrerkabine zwischen einer jeweiligen A-Säule und einer jeweiligen B-Säule erstrecken. Die Fahrerkabine umfasst weiterhin einen Fahrersitz, auf dem ein Maschinenführer der jeweiligen Arbeitsmaschine Platz nehmen kann.

Die Fahrerkabine umfasst des Weiteren ein Visualisierungssystem, mittels dessen Informationen für den Maschinenführer visualisierbar sind. Dies kann sowohl über Displays, beispielsweise in Form von LC- oder OLED-Displays, als auch über mindestens eine Scheibenanzeige erfolgen, beispielsweise in Form eines Head-up Displays. Die Scheibenanzeige kann beispielsweise mittels in Glasscheiben einlaminierter Displayfolien ausgeführt sein. Die Fahrerkabine umfasst weiterhin ein Überwachungssystem, mittels dessen Informationen zumindest über eine physische Verfassung des Maschinenführers erfassbar sind. Das Überwachungssystem kann beispielsweise dazu ausgebildet sein, eine Erschöpfung des Maschinenführers zu erkennen. Dies kann beispielsweise mittels der Verfolgung von Augenbewegungen, eine Erfassung von Schweißbildung an Händen oder an einer Stirn des Maschinenführers, einer Erkennung einer Sitzhaltung des Maschinenführers auf dem Fahrersitz, an einer Herzfrequenz und/oder einem Blutdruck des Maschinenführers oder dergleichen erfolgen. Weiterhin umfasst die Arbeitsmaschine eine Steuereinrichtung, die jeweils in Daten übertragender Weise mit dem Visualisierungssystem und dem Beobachtungssystem verbunden ist. Auf diese Weise ist die Steuereinrichtung dazu geeignet, Informationen von dem Beobachtungssystem zu empfangen und zu verarbeiten sowie das Visualisierungssystem anzusteuern.

Fahrerkabinen der vorstehend beschriebenen Art sind imstande Technik bereits bekannt. Hierzu wird beispielhaft auf das europäische Patent EP 2 712 754 B1 verwiesen. Dieses beschreibt ein Verfahren zum Betreiben einer Arbeitsmaschine, wobei die Betriebsweise der Arbeitsmaschine von einem emotionalen Zustand des Maschinenführers abhängig gemacht wird. Um diesen emotionalen Zustand zu erfassen, wird eine Interaktion des Maschinenführers mit mindestens einem Bedienelement ausgewertet. In Abhängigkeit dieser Auswertung wird automatisch ein Betriebsmodus aus einer Auswahl verschiedener Betriebsmodi aktiviert. Die Wahl des Betriebsmodus erfolgt dabei in solcher Weise, dass das Risiko einer Fehlbedienung durch den Maschinenführer minimiert wird.

Die US 2021/056306 A1 offenbart den Oberbegriff des Anspruchs 1.

Die beschriebene Vorgehensweise hat sich bereits als vorteilhaft herausgestellt. Allerdings ist ihre Zielsetzung auf die Minimierung von Fehlern ausgerichtet, nicht jedoch auf die Generierung einer möglichst hohen Zufriedenheit des Maschinenführers. Somit passt sich gemäß dem Stand der Technik die Arbeitsmaschine beispielsweise an eine schwindende Aufmerksamkeit des Maschinenführers an, unternimmt jedoch nichts, um die Aufmerksamkeit des Maschinenführers wieder zu steigern, wodurch eine höhere Arbeitsleistung generiert werden könnte, die am Ende eines jeweiligen Arbeitstages wiederum zu einer höheren Zufriedenheit des Maschinenführers beitragen würde.

Der vorliegenden Anmeldung liegt mithin die Aufgabe zugrunde, die Zufriedenheit eines Maschinenführers, die durch und/oder während eines Betriebs einer Arbeitsmaschine erreicht wird, zu steigern.

Die zugrunde liegende Aufgabe wird erfindungsgemäß mittels einer Fahrerkabine mit den Merkmalen des Anspruchs 1 gelöst. Vorteilhafte Ausgestaltungen ergeben sich aus den zugehörigen Unteransprüchen.

Die erfindungsgemäße Fahrerkabine ist dadurch gekennzeichnet, dass die Steuereinrichtung dazu eingerichtet ist, von dem Überwachungssystem erfasste Informationen zu verarbeiten und in Abhängigkeit der Informationen das Visualisierungssystem anzusteuern. Die Ansteuerung erfolgt in solcher Weise, das mittels des Visualisierungssystems mindestens eine Handlungsempfehlung visualisiert wird. Mit anderen Worten wird in Abhängigkeit der erfassten physischen Verfassung des Maschinenführers dem Maschinenführer automatisch mindestens eine Handlungsempfehlung visualisiert, die seiner physischen Verfassung Rechnung trägt. Somit ist es beispielsweise denkbar, dass mittels des Überwachungssystems verschiedene Parameter betreffend den Maschinenführer erfasst werden. Dies können je nach verwendeten Überwachungsmitteln beispielsweise eine Sitzhaltung des Maschinenführers in dem Fahrersitz oder Augenbewegungen des Maschinenführers sein. Weitere Möglichkeiten sind eingangs bereits erwähnt worden, wobei dies keine abschließende Auflistung darstellt. Die entsprechenden Informationen werden an die Steuereinrichtung übertragen und mittels dieser verarbeitet, wodurch die physische Verfassung des Maschinenführers ermittelt wird.

Wobei die Handlungsempfehlung vorgesehen und eingerichtet ist, die Aufmerksamkeit des Maschinenführers und kurzfristig oder langfristig die Produktivität des Maschinenführers zu erhöhen und wobei das Visualisierungssystem eine Scheibenanzeige zur Visualisierung der die Handlungsempfehlung umfassenden Information umfasst und die Information in einem Blickfeld des Maschinenführers visualisiert wird.

Für den beispielhaften Fall, dass das Ergebnis der Verarbeitung der Informationen des Überwachungssystems aussagt, dass eine Aufmerksamkeit des Maschinenführers vermindert ist, beispielsweise aufgrund von Langeweile, ist die Steuereinrichtung dazu eingerichtet, das Visualisierungssystem anzusteuern, sodass es eine Handlungsempfehlung visualisiert, die die Aufmerksamkeit des Maschinenführers wieder erhöhen soll. Beispielsweise ist es denkbar, dass dem Maschinenführer als Handlungsempfehlung die Aufnahme einer Aktivität vorgeschlagen wird, wobei diese Handlungsempfehlung mittels des Visualisierungssystems dem Maschinenführer visualisiert wird.

Die erfindungsgemäße Fahrerkabine hat viele Vorteile. Insbesondere ist sie dazu geeignet, den Maschinenführer mit Handlungsempfehlungen zu konfrontieren, die seiner aktuellen physischen Verfassung Rechnung tragen. Diese Handlungsempfehlungen sollen dazu beitragen, die physische Verfassung des Maschinenführers zu verbessern, wobei nach einer auf der Steuereinrichtung hinterlegten Logik in Abhängigkeit einer jeweils ermittelten physischen Verfassung bestimmte Handlungsempfehlungen visualisiert werden sollen. Diese Handlungsempfehlungen sind vorteilhafterweise darauf ausgerichtet, kurzfristig oder langfristig die Produktivität des Maschinenführers zu erhöhen, wodurch im Ergebnis seine Zufriedenheit maximiert werden soll. Dies kann beispielsweise auch darin bestehen, dem Maschinenführer eine Entspannung zu empfehlen, wenn beispielsweise aufkommende Müdigkeit erkannt wird. Eine Entspannungsphase kann dem Maschinenführer dabei helfen, zu einem späteren Zeitpunkt, zu dem es auf seine Aufmerksamkeit ankommt, in einer besseren physischen Verfassung zu sein, wodurch eine Leistungsfähigkeit des Maschinenführers erhöht und damit seine Zufriedenheit mit seinem Arbeitsergebnis erhöht werden.

In einer besonders vorteilhaften Ausgestaltung umfasst das Überwachungssystem eine tragbare Einheit, die der Maschinenführer tragen kann, insbesondere in Form eines am Handgelenk des Maschinenführers tragbaren Fitnesstrackers. Eine solche Einheit ist dazu geeignet, zumindest eine Herzfrequenz und/oder einen Blutdruck des Maschinenführers zu erfassen. Diese Informationen sind sodann an die Steuereinrichtung leitbar, mittels der sie in der vorstehend beschriebenen Weise verarbeitbar sind.

Weiterhin kann eine solche Fahrerkabine von besonderem Vorteil sein, bei der das Überwachungssystem eine Augenbeobachtung beinhaltet. Diese ist dazu geeignet, Informationen betreffend mindestens einen Augenparameter, insbesondere eine Pupillengröße eines jeweiligen Auges, und/oder eine Blickrichtung des Maschinenführers, zu erfassen. Ein solches Überwachungssystem kann insbesondere ein Überwachungsmittel in Form einer Kamera und/oder eines Eyetrackers umfassen.

Sofern das Überwachungssystem eine solche Augenbeobachtung beinhaltet, kann es weiterhin von besonderem Vorteil sein, wenn die Steuereinrichtung dazu eingerichtet ist, in Abhängigkeit von von dem Überwachungssystem erfassten Informationen betreffend eine Blickrichtung des Maschinenführers das Visualisierungssystem anzusteuern. Letzteres ist hierbei dazu eingerichtet, die Visualisierung mindestens einer Information der Blickrichtung des Maschinenführers nachzuführen. Mit anderen Worten ist die Steuereinrichtung in Zusammenwirkung mit dem Überwachungssystem und dem Visualisierungssystem bei dieser Ausgestaltung dazu geeignet, Informationen dem Maschinenführer immer so anzuzeigen, dass er sie in seinem Blickfeld hat, und zwar unabhängig von einer temporären Blickrichtung des Maschinenführers. Eine solche Ausgestaltung ist insbesondere dann vorteilhaft einsetzbar, wenn das Visualisierungssystem über mindestens eine mit einem lichtdurchlässigen Raumabschlusselement, insbesondere einer Glasscheibe, zusammenwirkende Scheibenanzeige verfügt, wie nachstehend gesondert erläutert ist.

Die erfindungsgemäße Fahrerkabine weiter ausgestaltend umfasst diese eine Sensoreinrichtung zur Erfassung von Informationen betreffend eine Ausrichtung des Fahrersitzes. Die Sensoreinrichtung ist mit der Steuereinrichtung in Daten übertragender Weise verbunden, sodass mittels der Sensoreinrichtung erfasste Informationen an die Steuereinrichtung leitbar sind. Die Steuereinrichtung ist dazu ausgebildet, die von der Sensoreinrichtung erfassten Informationen zu verarbeiten und in Abhängigkeit der Informationen das Visualisierungssystem anzusteuern. Auf diese Weise ist es möglich, die mittels des Visualisierungssystems visualisierte Informationen in Abhängigkeit der Ausrichtung des Fahrersitzes zu visualisieren, sodass im Ergebnis der Maschinenführer die Informationen stets in einem räumlichen Bereich visualisiert bekommt, in dessen Richtung sein Körper ausgerichtet ist. Das Visualisierungssystem ist bei dieser Ausgestaltung zu einer entsprechenden Nachführung der Visualisierungen eingerichtet.

In einer weiterhin vorteilhaften Ausgestaltung umfasst die Fahrerkabine mindestens ein Audiosystem, das zur akustischen Wiedergabe von Informationen für den Maschinenführer geeignet ist. Das Audiosystem ist hierbei mit der Steuereinrichtung in Daten übertragender Weise verbunden. Hierdurch ist die Möglichkeit geschaffen, mindestens eine Handlungsempfehlung zusätzlich oder alternativ zu einer Visualisierung mittels des Visualisierungssystems akustisch über das Audiosystem abzugeben. Auch ist eine kombinierte Verwendung des Visualisierungssystems und des Audiosystems in der Weise denkbar, dass bestimmte Anteile einer jeweilig an den Maschinenführer zu übermittelnden Informationen ausschließlich visuell mittels des Visualisierungssystems und andere Anteile ausschließlich akustisch mittels des Audiosystems übermittelt werden. Die Übermittlung von Informationen an den Maschinenführer über verschiedene Sinneswahrnehmungen ist besonders vorteilhaft, um eine Aufmerksamkeit des Maschinenführers zu erhöhen. Auch kann das Audiosystem von Vorteil sein, um in Abhängigkeit einer von dem Maschinenführer ausgewählten Handlungsempfehlung akustische Inhalte wiederzugeben. Hierbei kann es sich beispielsweise um akustische Anweisungen im Rahmen einer Fitnessübung oder um die Wiedergabe von Musik im Rahmen einer Entspannungsübung handeln.

In einer besonders vorteilhaften Ausgestaltung der erfindungsgemäßen Fahrerkabine ist die Steuereinrichtung in verschiedenen Arbeitsmodi betreibbar. Bei dieser Ausgestaltung ist die Steuereinrichtung dazu eingerichtet, das Visualisierungssystem in Abhängigkeit von einem jeweils aktiven Arbeitsmodus anzusteuern, sodass für den aktiven Arbeitsmodus relevante Informationen priorisiert visualisiert werden. Eine solche Priorisierung kann in einer veränderten Darstellungsweise, beispielsweise in der Vergrößerung grafischer Elemente und/oder einer Veränderung einer räumlichen Anordnung grafischer Elemente bestehen. Auch ist es denkbar, bestimmte Informationen nur dann anzuzeigen und umgekehrt andere Informationen auszublenden, wenn ein bestimmter Arbeitsmodus aktiv ist.

Insbesondere ist es denkbar, dass die Steuereinrichtung verschiedene Arbeitsmodi in Form eines Maschinenmodus und/oder eines Officemodus und/oder eines Entspannungsmodus umfasst. Diese unterscheiden sich dadurch, dass ein Fokus bei Betrieb der Steuereinrichtung in dem Maschinenmodus auf der Bedienung der Arbeitsmaschine, in dem Officemodus auf der Ausführung von Bürotätigkeiten und in dem Entspannungsmodus auf der Entspannung des Maschinenführers liegt.

Somit ist es insbesondere denkbar, dass bei Aktivierung des Maschinenmodus Informationen betreffend Betriebsparameter der Arbeitsmaschine oder betreffend Umweltparameter, die mittels Sensoreinrichtungen der Arbeitsmaschine erfasst werden, priorisiert dem Maschinenführer visualisiert werden. Dies kann beispielsweise mittels vergrößerter Darstellungen entsprechender grafischer Elemente oder grafischer Effekte, beispielsweise einer farblichen Absetzung oder dergleichen, erfolgen. Auch ist es denkbar, dass in dem Maschinenmodus eine im Vergleich zu anderen Arbeitsmodi größere Anzahl von Informationen visualisiert werden, sodass der Maschinenführer sich ein möglichst umfassendes Bild von einem aktuellen Betriebszustand der Arbeitsmaschine machen kann.

Die Visualisierung derartiger Informationen ist beispielsweise bei Aktivierung des Entspannungsmodus weniger sinnvoll, da letzterer mutmaßlich nur dann aktiviert wird, wenn der Maschinenführer sich in einer physischen Verfassung befindet, in der seine Konzentrationsfähigkeit vermindert ist, beispielsweise infolge einer Erschöpfung. Die Konfrontation des Maschinenführers mit einer Fülle von Informationen wäre in einer solchen Situation kontraproduktiv und verringert lediglich die Motivation und Zufriedenheit des Maschinenführers. Daher ist es weitaus sinnvoller, dem Maschinenführer bei aktiviertem Entspannungsmodus Informationen betreffend Betriebsparameter der Arbeitsmaschine lediglich reduziert zu visualisieren, während Informationen, die zur Entspannung des Maschinenführers beitragen können, visualisiert werden. Hierbei kann es sich beispielsweise um die Wiedergabe von Videos handeln.

Bei Aktivierung des Officemodus können dem Maschinenführer beispielsweise Informationen betreffend anfallende Bürotätigkeiten visualisiert werden, sodass diese bereits während der Arbeitszeit auf der Arbeitsmaschine gesichtet und ggf. bearbeitet werden können. Die Aktivierung des Officemodus kann insbesondere in Situationen angezeigt sein, in denen eine gesteigerte Aufmerksamkeit des Maschinenführers auf den Betrieb der Arbeitsmaschine nicht erforderlich ist, gleichwohl die Leistungsfähigkeit des Maschinenführers hoch ausgeprägt ist, sodass er zur Erledigung anderer Tätigkeiten mental und körperlich sowohl in der Lage als auch gewillt ist. Durch die Erledigung von Bürotätigkeiten empfindet der Maschinenführer die verbrachte Arbeitszeit als produktiver, wodurch unmittelbar die Zufriedenheit des Maschinenführers gesteigert wird.

Entsprechend der vorstehenden Erläuterung ist es mithin besonders vorteilhaft, wenn die Steuereinrichtung dazu eingerichtet ist, das Visualisierungssystem derart anzusteuern, dass eine Visualisierungsstruktur visualisierter Informationen veränderbar ist bzw. verändert wird, wobei mindestens eine Information einblendbar und oder eine andere Information ausblendbar sind. Entsprechende Beispiele sind vorstehend bereits genannt. Die Veränderung der Visualisierungsstruktur kann ebenso in der Änderung einer Anordnung grafischer Elemente sowie einer Veränderung von Größen grafischer Elemente erfolgen. Auch ist eine Änderung der Gestaltung grafischer Elemente denkbar.

Die erfindungsgemäße Fahrerkabine weiter ausgestaltend umfasst das Visualisierungssystem mindestens eine Scheibenanzeige, die mit der Frontscheibe und/oder einer Seitenscheibe zusammenwirkt. Derartige Scheibenanzeigen sind grundsätzlich von Head-up Displays bekannt, wobei vorzugsweise mittels eines Projektors ein Bild auf die jeweilige Scheibe projiziert wird oder in die Scheibe eine Displayfolie einlaminiert ist. Die Scheibenanzeige ist dazu geeignet, Informationen in oder auf der jeweiligen Scheibe zu visualisieren. Die Ausgestaltung des Visualisierungssystems in dieser Art und Weise hat den besonderen Vorteil, dass die jeweiligen Informationen stets unmittelbar in einem Blickfeld des Maschinenführers visualisiert werden können.

Weiterhin kann eine solche Ausgestaltung von Vorteil sein, bei der das Visualisierungssystem mindestens ein Display umfasst. Insbesondere kann es von Vorteil sein, wenn mindestens ein Display an einer Tragsäule angeordnet ist, vorzugsweise in diese integriert ist. Hierbei ist es besonders von Vorteil, wenn jeweils ein Display an zwei von A-Säulen gebildeten Tragsäulen einer jeweiligen Tragstruktur der Fahrkabine angeordnet sind. Mittels derartiger Displays, die insbesondere von LC- oder OLED-Displays gebildet sein können, verfügt das Visualisierungssystem über ausreichend Visualisierungsfläche, um dem Maschinenführer Informationen in ausreichendem Umfang zu visualisieren. Hierbei kann es besonders von Vorteil sein, wenn das Visualisierungssystem dazu eingerichtet ist, bestimmte Arten von Informationen in einer vorgegebenen Visualisierungsstruktur stets mittels des mindestens einen Displays zu visualisieren, während andere Arten von Informationen stets mittels einer anderen Einheit des Visualisierungssystems, insbesondere mittels einer Scheibenanzeige oder eines anderen Displays, visualisiert werden. Auf diese Weise kann sich der Maschinenführer an eine bestimmte Art und Weise der Visualisierung gewöhnen, wodurch ihm ein Überblick über die Informationen und mithin die Bedienung der Arbeitsmaschine erleichtert werden. Hierbei kann es weiterhin von besonderem Vorteil sein, wenn das Visualisierungssystem dazu eingerichtet ist, dass der Maschinenführer die Visualisierungsstruktur gemäß persönlichen Vorlieben konfigurieren kann.

In einer besonders vorteilhaften Ausgestaltung der erfindungsgemäßen Fahrerkabine umfasst diese mindestens eine Eingabeeinheit, die in Daten übertragender Weise mit der Steuereinheit verbunden ist. Mittels der Eingabeeinheit ist die Möglichkeit geschaffen, dass der Maschinenführer Eingaben vornimmt, die sodann an die Steuereinheit geleitet und mittels dieser verarbeitet werden. In besonders bevorzugter Weise umfasst die Eingabeeinheit mindestens ein an einer Armlehne des Fahrersitzes angeordnetes Bedienelement, beispielsweise in Form einer Tastatur oder eines Multifunktion-Joysticks. Auch kann es von Vorteil sein, wenn der Fahrersitz über ein Tischelement verfügt, das schwenkbar relativ zu einer Sitzfläche des Fahrersitzes ausgebildet ist. Mindestens ein Bedienelement, insbesondere eine Tastatur, kann an dem Tischelement angeordnet, vorzugsweise in dieses integriert sein, wodurch die Eingabe dem Maschinenführer die Eingabe von Daten oder Befehlen besonders erleichtert wird.

Weiterhin kann es von besonderem Vorteil sein, wenn dem Fahrersitz ein Antriebssystem zugeordnet ist, mittels dessen der Fahrersitz motorisch verstellbar ist. Das Antriebssystem ist in Daten übertragender Weise mit der Steuereinrichtung verbunden, sodass der Fahrersitz mittels einer Ansteuerung des Antriebssystems verstellbar ist. Mittels einer derart ausgestalteten Fahrerkabine ist es beispielsweise möglich, den Fahrersitz in Abhängigkeit der physischen Verfassung des Maschinenführers automatisch zu verstellen. Beispielsweise ist es denkbar, dass der Maschinenführer aufgrund einer ihm unterbreiteten Handlungsempfehlung infolge einer erkannten physischen Verfassung, die auf eine Erschöpfung schließen lässt, die Steuereinrichtung in einem Entspannungsmodus betreibt. Die Aktivierung des Entspannungsmodus kann damit verbunden sein, das Antriebssystem des Fahrersitzes anzusteuern, sodass der Fahrersitz in eine Entspannungsstellung bewegt wird. Diese kann sich beispielsweise durch eine stärkere Neigung einer Rückenlehne des Fahrersitzes auszeichnen, wodurch dem Maschinenführer zu einer entspannten Sitzposition verholfen wird. Analog ist es denkbar, dass der Fahrersitz im Zuge der Durchführung andere Aktionen automatisch mittels Ansteuerung durch die Steuereinrichtung verstellt wird. Die Verarbeitung der erfassten Informationen betreffend die physische Verfassung des Maschinenführers ist in einem solchen Fall denkbar.

Die zugrunde liegende Aufgabe wird außerdem mittels einer landwirtschaftlichen Arbeitsmaschine mit den Merkmalen des Anspruchs 17 gelöst. Diese Arbeitsmaschine, die beispielsweise von einem Schlepper oder von einer landwirtschaftlichen Erntemaschine, insbesondere einem Mähdrescher, gebildet sein kann, umfasst erfindungsgemäß eine Fahrerkabine gemäß einer der vorstehend beschriebenen Ausgestaltungen. Die sich hierdurch ergebenden Vorteile sind vorstehend bereits dargelegt. Insbesondere wird erreicht, dass dem Maschinenführer der Arbeitsmaschine in Abhängigkeit von seiner physischen Verfassung Handlungsempfehlungen visualisiert werden. Die Handlungsempfehlungen basieren auf der grundsätzlichen Idee, eine Aktivität des Maschinenführers dessen physischer Verfassung anzupassen, sodass eine Zufriedenheit des Maschinenführers gesteigert wird.

Schließlich wird die zugrunde liegende Ausgabe mittels eines Verfahrens zum Betrieb einer landwirtschaftlichen Arbeitsmaschine mit den Merkmalen des Anspruchs 18 gelöst. Das Verfahren ist dadurch gekennzeichnet, dass die Steuereinrichtung in Abhängigkeit der von dem Überwachungssystem erfassten Informationen betreffend die physische Verfassung des Maschinenführers das Visualisierungssystem ansteuert, sodass das Visualisierungssystem mindestens eine Handlungsempfehlung visualisiert. Die sich hierdurch ergebenden Vorteile sind vorstehend bereits dargelegt.

Die Erfindung ist nachstehend anhand eines Ausführungsbeispiels, das in den Figuren dargestellt ist, näher erläutert. Es zeigt:
- Fig. 1:: Einen Querschnitt durch eine erfindungsgemäße Arbeitsmaschine in Form eines Mähdreschers,
- Fig. 2:: Eine perspektivische Ansicht einer Fahrerkabine der Arbeitsmaschine gemäß Figur 1,
- Fig. 3:: Ein Detail eines Fahrersitzes der Arbeitsmaschine gemäß Figur 1,
- Fig. 4:: Eine Ansicht eines Frontteils der Fahrerkabine gemäß Figur 2, wobei mittels eines Visualisierungssystems Informationen visualisiert sind,
- Fig. 5:: Ein Detail eines Displays, mittels dessen Informationen visualisiert sind,
- Fig. 6:: Ein Detail eines weiteren Displays, mittels dessen Informationen visualisiert sind und
- Fig. 7:: Ein weiteres Detail des Displays gemäß Figur 5, mittels dessen Informationen visualisiert sind.

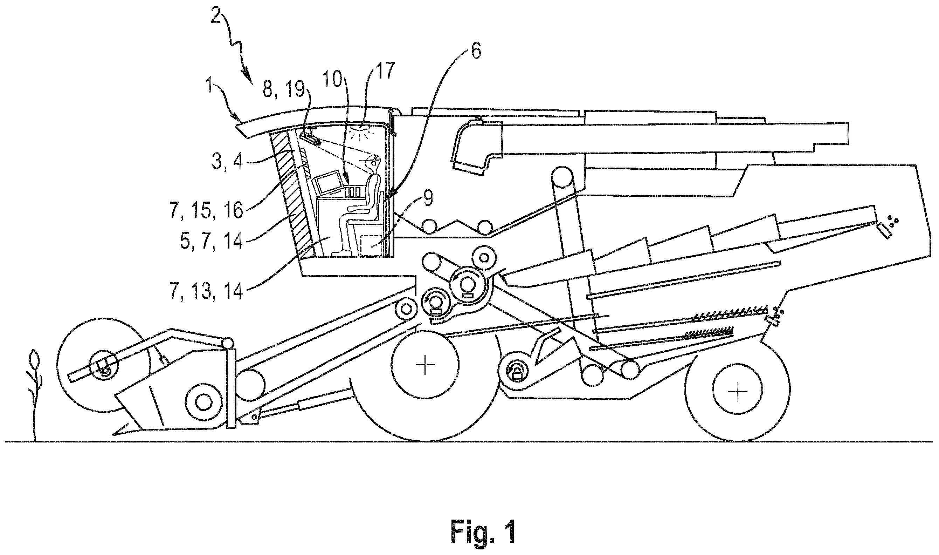

Ein Ausführungsbeispiel, das in den **Figuren 1 bis 7** dargestellt ist, umfasst eine Arbeitsmaschine **2,** die mit einer erfindungsgemäßen Fahrerkabine **1** ausgestattet ist. Bei der Arbeitsmaschine **2** handelt es sich in dem gezeigten Beispiel um einen Mähdrescher, dessen grundsätzlicher Aufbau bekannt ist. Somit umfasst die Arbeitsmaschine **2** eine Mehrzahl verschiedener Arbeitsorgane, derer es zur Ernte jeweiliger Pflanzen sowie der Separierung darin enthaltener Früchte bedarf.

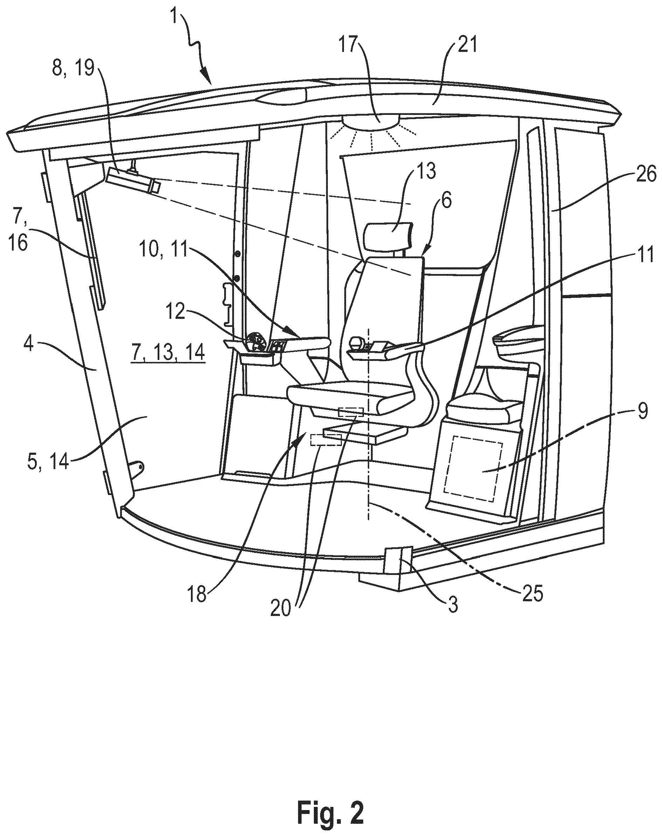

Zur Steuerung sowie allgemeinen Überwachung eines Betriebs der Arbeitsmaschine **2** umfasst selbige die genannte Fahrerkabine **1,** in der ein Maschinenführer auf einem Fahrersitz **6** Platz nehmen kann. Die Fahrerkabine 1 ergibt sich besonders gut anhand von **Figur** 2. Ferner umfasst die Fahrerkabine **1** eine Eingabeeinheit **10,** mittels der der Maschinenführer Eingaben, insbesondere solche betreffend einen Betrieb der Arbeitsmaschine **2,** vornehmen kann. Ferner umfasst die Fahrerkabine 1 eine Tragstruktur, die in dem gezeigten Beispiel ein Dach **21** sowie zwei Tragsäulen **3, 4** umfasst, die A-Säulen der Tragstruktur bilden. Zwischen den Tragsäulen **3, 4** erstreckt sich in Querrichtung der Arbeitsmaschine **1** eine Frontscheibe **5,** die einen vorderen räumlichen Abschluss der Fahrerkabine **1** bildet. Ferner umfasst die Fahrerkabine **1** an ihren Seiten jeweils eine Seitenscheibe **13,** wobei sich die Seitenscheiben **13** zwischen einer jeweiligen Tragsäule **3, 4** und einer zugeordneten hinteren Tragsäule **26,** die von einer B-Säule gebildet ist, erstrecken.

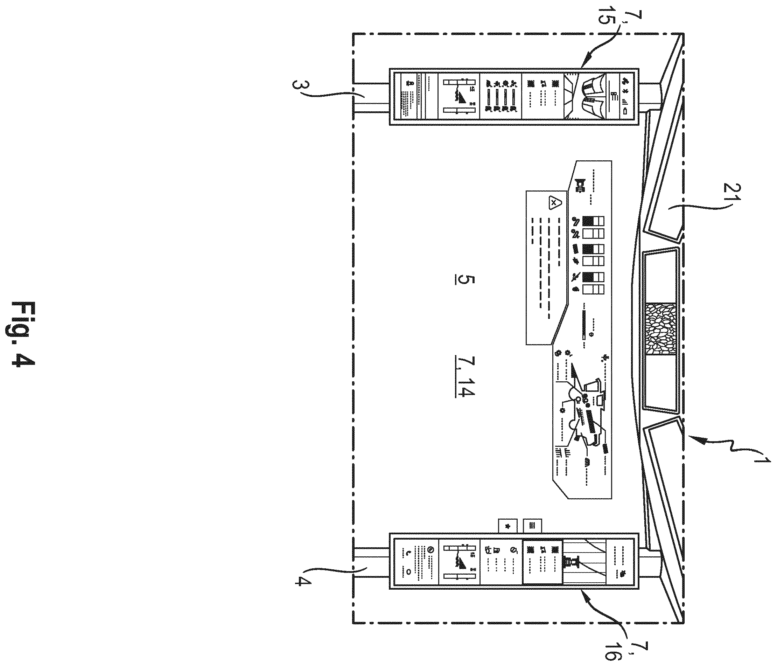

Die Fahrerkabine **1** umfasst ferner ein Visualisierungssystem **7,** mittels dessen dem Maschinenführer Informationen visualisierbar sind. In dem gezeigten Beispiel umfasst das Visualisierungssystem **7** mehrere Komponenten, nämlich insbesondere zwei Displays **15, 16,** von denen jeweils eine einer der beiden Tragsäulen **3, 4** zugeordnet ist. Die Displays **15, 16** sind hier jeweils von LC-Displays gebildet. Dies ergibt sich besonders gut anhand von **Figur 4****.** Ferner umfasst das Visualisierungssystem **7** in dem gezeigten Beispiel eine Scheibenanzeige **14,** die hier mehrere Displayfolien umfasst, von denen jeweils eine in die Frontscheibe **5** sowie jeweils eine in die Seitenscheiben **13** einlaminiert sind. Die Displays **15, 16** sowie die Scheibenanzeige **14** sind dazu geeignet, dem Maschinenführer Informationen zu visualisieren, wobei insbesondere die Darstellung von grafischen Elementen **22, 23, 24** möglich ist.

Ferner umfasst die Fahrerkabine **1** ein Überwachungssystem **8,** das dazu geeignet ist, Informationen betreffend eine physische Verfassung des Maschinenführers zu erfassen. In dem gezeigten Beispiel umfasst das Überwachungssystem **8** ein Überwachungsmittel in Form einer Kamera **19,** die an einem vorderen Ende der Fahrerkabine 1 angeordnet und auf einen Kopfbereich eines auf dem Fahrersitz 6 sitzenden Maschinenführers ausgerichtet ist. Mittels der Kamera **19** ist es möglich, mindestens einen Augenparameter eines Maschinenführers zu beobachten und hierüber entsprechende Informationen zu sammeln.

Sowohl das Visualisierungssystem **7** als auch das Überwachungssystem **8** sind jeweils in Daten übertragender Weise mit einer Steuereinrichtung **9** verbunden, die gleichermaßen Teil der Fahrerkabine **1** ist. Die Steuereinrichtung **9,** die insbesondere eine Datenverarbeitungsanlage umfassen kann, ist dazu eingerichtet, Daten zu empfangen, zu speichern, unter Ausführung von Software zu verarbeiten und automatisiert Steuerbefehle an das Visualisierungssystem **7** zu senden. Insbesondere ist die Steuereinrichtung **9** dazu eingerichtet, die mittels des Überwachungssystems **8** erfassten Informationen zu verarbeiten und hieraus auf die physische Verfassung des Maschinenführers zu schließen. Sodann ist die Steuereinrichtung **9** dazu eingerichtet, in Abhängigkeit der ermittelten physischen Verfassung des Maschinenführers automatisch das Visualisierungssystem **7** anzusteuern, sodass mittels des Visualisierungssystems **7** Handlungsempfehlungen visualisiert werden. In dem gezeigten Beispiel ist die Steuereinrichtung **9** dazu eingerichtet, in drei unterschiedlichen Arbeitsmodi betrieben zu werden. Hierbei handelt es sich um einen Maschinenmodus, einen Officemodus und einen Entspannungsmodus.

In dem Maschinenmodus, der hier beispielhaft gemäß **Figur 4** veranschaulicht ist, liegt der Fokus des Betriebs auf der Bedienung der Arbeitsmaschine **2.** Entsprechend ist die Steuereinrichtung **9** dazu eingerichtet, das Visualisierungssystem **7** dahingehend anzusteuern, dass dem Maschinenführer hauptsächlich Informationen betreffend einen jeweiligen Betriebszustand der Arbeitsmaschine **2** visualisiert werden. Dies kann insbesondere mittels der Displays **15, 16** sowie der Scheibenanzeige **14** erfolgen. In dem Maschinenmodus werden in dem gezeigten Beispiel Informationen betreffend einen Betriebszustand der Arbeitsmaschine 1 in größerer Anzahl sowie einzelne Informationen vergrößert dargestellt, um dem Maschinenführer ein möglichst vollständiges Bild von dem Betriebszustand zu verschaffen.

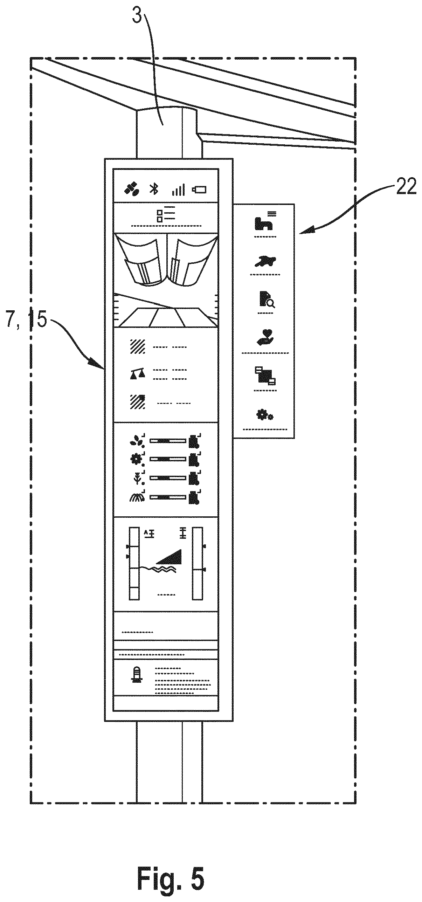

Sofern die Verarbeitung der Informationen, die von dem Überwachungssystem **8** erfasst wurden, zu dem Schluss führt, dass sich der Maschinenführer in einer erschöpften Verfassung befindet, steuert die Steuereinrichtung **9** das Visualisierungssystem **7** dahingehend an, dass dem Maschinenführer eine Handlungsempfehlung visualisiert wird. Dies kann beispielsweise mittels der Visualisierung eines Auswahlmenüs erfolgen, wie es anhand eines grafisches Elements **22** gemäß der Darstellung in **Figur 5** veranschaulicht ist.

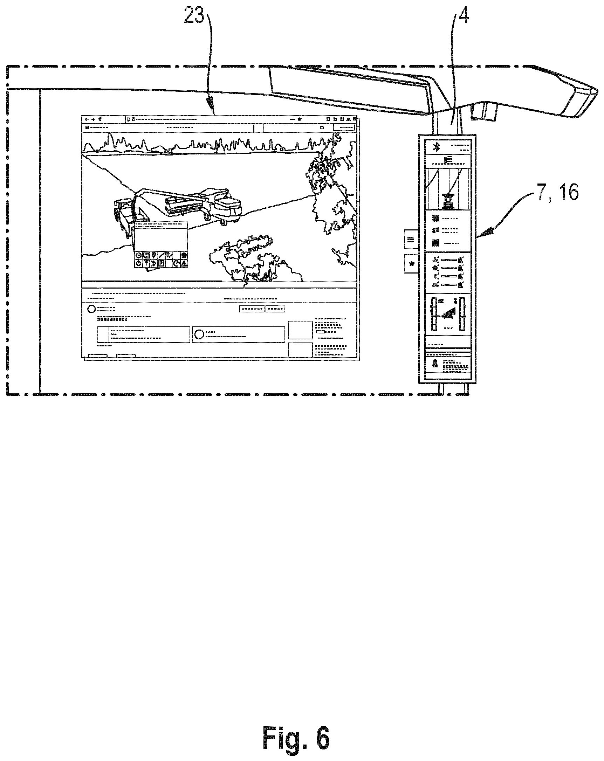

Beispielsweise kann dem Maschinenführer die Handlungsempfehlung visualisiert werden, sein Wohlbefinden zu verbessern. Der Maschinenführer kann in diesem Fall mittels der Eingabeeinheit **10,** insbesondere mittels eines an einer Armlehne **11** angeordneten Bedienelements **12** der Eingabeeinheit **10,** die Handlungsempfehlung annehmen bzw. auswählen, woraufhin dem Maschinenführer beispielsweise verschiedene Optionen visualisiert werden können, auf welche Weise der Maschinenführer sein Wohlbefinden verbessern möchte. Beispielsweise ist denkbar, dass der Maschinenführer die Wiedergabe von Musik wählt, woraufhin über ein Audiosystem **17** entsprechende Musik wiedergegeben wird. Die Steuereinrichtung **9** wird in diesem Fall bis auf Weiteres in ihrem Entspannungsmodus betrieben. Auch ist es denkbar, dass mittels des Visualisierungssystems **7** mindestens ein grafisches Element **23** visualisiert wird, in dem ein Internetbrowser wiedergegeben ist. Dies ergibt sich beispielhaft aus **Figur 6****.**

In dem gezeigten Beispiel ist die Steuereinrichtung **9** dazu eingerichtet, das Visualisierungssystem **7** dahingehend anzusteuern, dass eine Visualisierungsstruktur der visualisierten Informationen verändert wird. In dem skizzierten Fall wird die Steuereinrichtung **9** mithin das Visualisierungssystem **7** ansteuern, sodass während des Betriebs der Steuereinrichtung **9** in dem Entspannungsmodus die Visualisierung von Informationen betreffend einen Betriebszustand der Arbeitsmaschine **2** lediglich vermindert visualisiert werden. Dies kann sowohl eine reduzierte Visualisierung umfassen, bei der die entsprechenden Informationen in kleineren grafischen Elementen dargestellt werden, als auch ein vollständiges Auslassen von Informationen, deren weitere Darstellung dem kurzfristigen Ziel der Entspannung des Maschinenführers zuwiderlaufen würden.

In einer anderen Konstellation ist es denkbar, dass der Maschinenführer sich in einer besonders wachen und leistungsfähigen Verfassung befindet. In einer solchen Situation ist es sinnvoll, das Leistungspotenzial des Maschinenführers auszuschöpfen, wodurch wiederum seine Zufriedenheit gesteigert wird. In diesem Fall wird die Steuereinrichtung **9** in dem Maschinenmodus betrieben, in dem sie das Visualisierungssystem **7** ansteuert, Betriebsparameter der Arbeitsmaschine **2** sowie etwaige Umweltparameter, die mittels nicht näher dargestellten Sensoreinrichtungen der Arbeitsmaschine **2** erfasst werden können, vermehrt darzustellen, sodass die Maschinenführer ein möglichst umfassender Überblick über den Betriebszustand der Arbeitsmaschine **2** sowie äußerer Randbedingungen des jeweiligen Arbeitseinsatzes vermittelt werden.

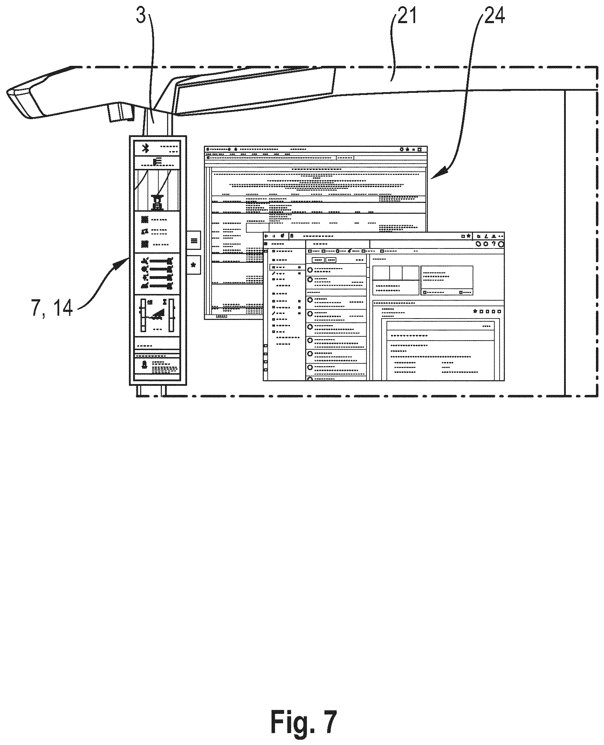

Sofern eine jeweilige Arbeitssituation der Arbeitsmaschine **2** eine gesteigerte Aufmerksamkeit des Maschinenführers nicht erfordert, er jedoch gleichwohl in einer physischen Verfassung ist, in der er leistungsfähig und motiviert ist, wird die Steuereinrichtung **9** das Visualisierungssystem **7** dahingehend ansteuern, dem Maschinenführer die Ausführung von Office-Arbeiten zu empfehlen. Sofern der Maschinenführer dem folgt, wird die Steuereinrichtung **9** in ihrem Officemodus betrieben. Hierzu ist es insbesondere denkbar, dass mittels des Visualisierungssystems **7** grafische Elemente **24** visualisiert werden, die Informationen betreffend Bürotätigkeiten des Maschinenführers beinhalten. Dieses beispielhaft anhand von **Figur 7** veranschaulicht.

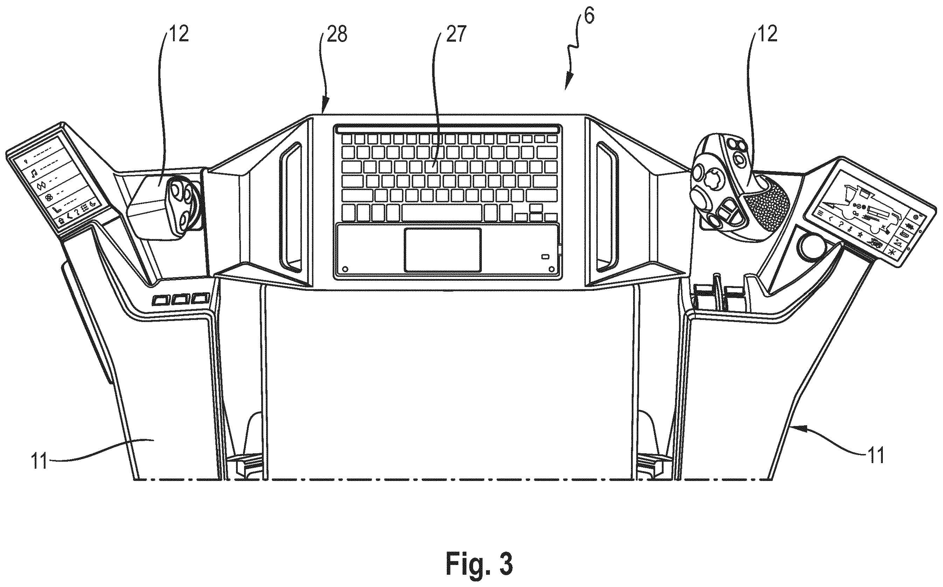

In dem Officemodus steuert die Steuereinrichtung 9 das Visualisierungssystem **7** dahingehend an, dass Informationen betreffend den Betriebszustand der Arbeitsmaschine **2** reduziert visualisiert werden, während Informationen betreffend anstehende Bürotätigkeiten des Maschinenführers visualisiert werden. Diese erlauben es nunmehr dem Maschinenführer, mittels der Eingabeeinheit **10** Eingaben vorzunehmen und auf diese Weise die Bürotätigkeiten zu erledigen, die ansonsten nach Beendigung des Arbeitseinsatzes mit der Arbeitsmaschine **2** erledigt werden müssten. Dem Maschinenführer wird mithin die Möglichkeit eingeräumt, seine Arbeitszeit optimal zu nutzen, wodurch eine Zufriedenheit gesteigert wird. Die Eingabeeinheit **10** kann zu diesem Zweck insbesondere eine Tastatur **27** umfassen, die in ein Tischelement **28** des Fahrersitzes **6** integriert ist. Dies ergibt sich besonders gut anhand von **Figur 3****.**

In besonders vorteilhafter Weise umfasst das Überwachungssystem **8** in dem gezeigten Beispiel einen nicht näher dargestellten Eyetracker, mittels dessen Informationen betreffend Augenbewegungen des Maschinenführers erfassbar sind. Die Verarbeitung dieser Informationen mittels der Steuereinrichtung **9** ermöglicht es der Steuereinrichtung **9,** das Visualisierungssystem **7** dahingehend anzusteuern, dass visualisierte Informationen derart nachgeführt werden, dass sie sich zumindest vornehmlich in einer jeweils aktuellen Blickrichtung des Maschinenführers befinden. Auf diese Weise ist die Bedienung der Arbeitsmaschine **2** für den Maschinenführer besonders bequem, da jeweils relevante Informationen stets in seinem Blickfeld visualisiert werden.

Ferner ist die Fahrerkabine **1** in dem gezeigten Beispiel in besonders bevorzugter Weise mit einem Antriebssystem **18** ausgestattet, das dem Fahrersitz **6** zugeordnet ist. Das Antriebssystem **18** ist dazu geeignet, den Fahrersitz 6 mittels mehrerer Antriebsmotoren **20** motorisch zu verstellen, wobei das Antriebssystem **18** mittels der Steuereinrichtung **9** ansteuerbar ist. Auf diese Weise kann die Steuereinrichtung **9** eine Stellung des Fahrersitzes **6** in Abhängigkeit beispielsweise eines jeweils aktiven Arbeitsmodus verändern. Bei Betrieb in dem Entspannungsmodus kann beispielsweise eine Neigung einer Rückenlehne des Fahrersitzes **6** automatisch mittels des Antriebssystems **18** geändert werden, sodass der Maschinenführer eine entspannte Sitzposition einnehmen kann. Bei Aktivierung des Officemodus kann der Fahrersitz **6** hingegen eine besonders aufrechte Stellung verstellt werden, die dem Maschinenführer eine auf die Erledigung von Bürotätigkeit hin optimierte Sitzposition erlaubt.

Ferner wirkt der Fahrersitz **6** in dem gezeigten Beispiel mit einer Sensoreinrichtung **26** zusammen, die dazu geeignet ist, Informationen betreffend eine Stellung des Fahrersitzes **6** zu erfassen. Diese Informationen sind an die Steuereinrichtung **9** leitbar, die hierzu in Daten übertragender Weise mit der Sensoreinrichtung **26** verbunden ist. Steuereinrichtung **9** ist dazu eingerichtet, die auf diese Weise erfassten Informationen zu verarbeiten und in Abhängigkeit davon das Visualisierungssystem **7** anzusteuern. Dies erfolgt unter der Zielsetzung, die mittels des Visualisierungssystems **7** visualisierte Informationen der jeweiligen Stellung des Fahrersitzes **6** nachzuführen. Hierdurch ist die Möglichkeit geschaffen, insbesondere bei einer Veränderung einer Drehstellung des Fahrersitzes **6** um eine Drehachse **25,** beispielsweise ausgehend von einer auf die Frontscheibe **5** ausgerichteten Stellung hin zu einer auf eine Seitenscheibe **13** ausgerichteten Stellung, die visualisierte Informationen nachzuführen und mithin nicht länger mittels der Scheibenanzeige **14** in der Frontscheibe **5,** sondern stattdessen in der jeweiligen Seitenscheibe **13** dem Maschinenführer zu visualisieren. Auf diese Weise hat der Maschinenführer die jeweiligen Informationen stets in seinem Blickfeld kann sie entsprechend anstrengungsfrei aufnehmen und würdigen.

### Bezugszeichenliste

- 1: Fahrerkabine
- 2: Arbeitsmaschine
- 3: Tragsäule
- 4: Tragsäule
- 5: Frontscheibe
- 6: Fahrersitz
- 7: Visualisierungssystem
- 8: Überwachungssystem
- 9: Steuereinrichtung
- 10: Eingabeeinheit
- 11: Armlehne
- 12: Bedienelement
- 13: Seitenscheibe
- 14: Scheibenanzeige
- 15: Display
- 16: Display
- 17: Audiosystem
- 18: Antriebssystem
- 19: Kamera
- 20: Antriebsmotor
- 21: Dach
- 22: grafisches Element
- 23: grafisches Element
- 24: grafisches Element
- 25: Drehachse
- 26: Tragsäule
- 27: Tastatur
- 28: Tischelement

## Patentansprüche

1. Fahrerkabine (1) für eine landwirtschaftliche Arbeitsmaschine (2), umfassend
- eine sich zwischen zwei Tragsäulen (3, 4) erstreckende Frontscheibe (5),
- einen Fahrersitz (6),
- mindestens ein Visualisierungssystem (7) zur Visualisierung von Informationen für einen Maschinenführer,
- ein Überwachungssystem (8) zur Erfassung von Informationen betreffend eine physische Verfassung des Maschinenführers sowie
- eine Steuereinrichtung (9), die mit dem Visualisierungssystem (7) und dem Überwachungssystem (8) jeweils in Daten übertragender Weise verbunden ist,
**dadurch gekennzeichnet, dass**
die Steuereinrichtung (9) dazu eingerichtet ist, von dem Überwachungssystem (8) erfasste Informationen zu verarbeiten und in Abhängigkeit der Informationen das Visualisierungssystem (7) anzusteuern, sodass mittels des Visualisierungssystems (7) mindestens eine Handlungsempfehlung visualisiert wird, wobei die Handlungsempfehlung vorgesehen und eingerichtet ist, die Aufmerksamkeit des Maschinenführers und kurzfristig oder langfristig die Produktivität des Maschinenführers zu erhöhen und wobei das Visualisierungssystem (7) eine Scheibenanzeige (14) zur Visualisierung der die Handlungsempfehlung umfassenden Information umfasst und die Information in einem Blickfeld des Maschinenführers visualisiert wird.

2. Fahrerkabine nach einem der vorhergehenden Ansprüche das Überwachungssystem (8) eine tragbare Einheit umfasst, die der Maschinenführer tragen kann, insbesondere in Form eines am Handgelenk tragbaren Fitnesstrackers, mittels dessen zumindest eine Herzfrequenz und/oder ein Blutdruck des Maschinenführers erfassbar ist.

3. Fahrerkabine (1) nach einem der vorhergehenden Ansprüche, **dadurch**
**gekennzeichnet, dass** das Überwachungssystem (8) eine Augenbeobachtung beinhaltet, mit der Informationen betreffend mindestens einen Augenparameter, insbesondere eine Pupillengröße eines jeweiligen Auges, und/oder eine Blickrichtung des Maschinenführers, erfassbar sind.

4. Fahrerkabine (1) nach Anspruch 3, **dadurch gekennzeichnet, dass** die Steuereinrichtung (9) dazu eingerichtet ist, in Abhängigkeit von von dem Überwachungssystem (8) erfassten Informationen betreffend die Blickrichtung des Maschinenführers das Visualisierungssystem (7) anzusteuern, wobei das Visualisierungssystem (7) dazu eingerichtet ist, die Visualisierung mindestens einer Information der Blickrichtung des Maschinenführers nachzuführen.

5. Fahrerkabine (1) nach einem der vorhergehenden Ansprüche, **gekennzeichnet durch** eine Sensoreinrichtung (26) zur Erfassung von Informationen betreffend eine Stellung des Fahrersitzes (6), wobei die Steuereinrichtung (9) mit der Sensoreinrichtung (26) in Daten übertragender Weise verbunden sowie dazu eingerichtet ist, von der Sensoreinrichtung (26) erfasste Informationen zu verarbeiten und Abhängigkeit der Informationen das Visualisierungssystem (7) anzusteuern, sodass mittels des Visualisierungssystems (7) visualisierte Informationen einer Stellung des Fahrersitzes (6) nachgeführt werden.

6. Fahrerkabine (1) nach einem der vorhergehenden Ansprüche, **gekennzeichnet durch** mindestens ein Audiosystem (17) zur akustischen Wiedergabe von Informationen für den Maschinenführer, wobei das Audiosystem (17) mit der Steuereinrichtung (9) in Daten übertragender Weise verbunden ist.

7. Fahrerkabine (1) nach Anspruch 6, **dadurch gekennzeichnet, dass** die Steuereinrichtung (9) dazu eingerichtet ist, in Abhängigkeit der von dem Überwachungssystem (8) erfassten Informationen das Audiosystem (17) anzusteuern, sodass mittels des Audiosystems (17) mindestens eine Handlungsempfehlung akustisch wiedergegeben wird.

8. Fahrerkabine (1) nach einem der vorhergehenden Ansprüche, **dadurch**
**gekennzeichnet, dass** die Steuereinrichtung (9) in verschiedenen Arbeitsmodi betreibbar ist, wobei die Steuereinrichtung (9) dazu eingerichtet ist, das Visualisierungssystem (7) in Abhängigkeit von einem jeweils aktiven Arbeitsmodus anzusteuern, sodass für den aktiven Arbeitsmodus relevante Informationen priorisiert visualisiert werden.

9. Fahrerkabine (1) nach Anspruch 8, **dadurch gekennzeichnet, dass** mindestens ein Arbeitsmodus von einem Maschinenmodus und/oder einem Officemodus und/oder einem Entspannungsmodus gebildet ist, wobei ein Fokus in dem Arbeitsmodus auf der Bedienung der Arbeitsmaschine (2), in dem Officemodus auf der Ausführung von Bürotätigkeiten und in dem Entspannungsmodus auf der Entspannung des Maschinenführers liegt.

10. Fahrerkabine (1) nach Anspruch 8 oder 9, **dadurch gekennzeichnet, dass** die Steuereinrichtung (9) dazu eingerichtet ist, das Visualisierungssystem (7) in Abhängigkeit eines gewählten Arbeitsmodus anzusteuern, sodass eine Visualisierungsstruktur visualisierter Informationen verändert wird, wobei vorzugsweise mindestens eine Information vergrößert und/oder mindestens eine Information ausgeblendet und/oder mindestens eine Information in einem Blickfeld des Maschinenführers visualisiert wird.

11. Fahrerkabine (1) nach einem der Ansprüche 8 bis 10, **dadurch gekennzeichnet, dass** die Steuereinrichtung (9) dazu eingerichtet ist, das Visualisierungssystem (7) derart anzusteuern, dass eine Visualisierungsstruktur visualisierter Informationen veränderbar ist, wobei mindestens eine Information einblendbar und/oder eine andere Information ausblendbar ist.

12. Fahrerkabine (1) nach einem der vorhergehenden Ansprüche, **dadurch**
**gekennzeichnet, dass** das Visualisierungssystem (7) mindestens eine mit der Frontscheibe (5) und/oder einer Seitenscheibe (13) zusammenwirkende Scheibenanzeige (14), insbesondere in Form einer in die Frontscheibe (5) oder die Seitenscheibe (13) einlaminierten Displayfolie, umfasst, mittels der Informationen visualisierbar sind.

13. Fahrerkabine (1) nach einem der vorhergehenden Ansprüche, **dadurch**
**gekennzeichnet, dass** das Visualisierungssystem (7) mindestens ein Display (15, 16), insbesondere in Form eines LC-Displays, umfasst, die an einer Tragsäule (3, 4) angeordnet ist, vorzugsweise in eine Tragsäule integriert ist.

14. Fahrerkabine (1) nach einem der vorhergehenden Ansprüche, **gekennzeichnet durch** mindestens eine mit der Steuereinheit (9) wirkverbundene Eingabeeinheit (10), vorzugsweise in Form mindestens eines an einer Armlehne (11) des Fahrersitzes (6) angeordneten Bedienelements (12), mittels dessen von dem Maschinenführer Eingaben vornehmbar sind.

15. Fahrerkabine (1) nach einem der vorhergehenden Ansprüche, **dadurch**
**gekennzeichnet, dass** dem Fahrersitz (6) ein Antriebssystem (18) zugeordnet ist, mittels dessen der Fahrersitz (6) motorisch verstellbar ist, wobei das Antriebssystem (18) mit der Steuereinrichtung (9) in Daten übertragender Weise verbunden ist, sodass der Fahrersitz (6) mittels einer Ansteuerung des Antriebssystems (18) verstellbar ist.

16. Fahrerkabine (1) nach den Ansprüchen 9 und 15, **dadurch gekennzeichnet, dass** die Steuereinrichtung (9) dazu eingerichtet ist, das Antriebssystem (18) in Abhängigkeit eines aktiven Arbeitsmodus anzusteuern.

17. Landwirtschaftliche Arbeitsmaschine (2),
**gekennzeichnet durch**
eine Fahrerkabine (1) nach einem der vorhergehenden Ansprüche.

18. Verfahren zum Betrieb einer landwirtschaftlichen Arbeitsmaschine (2), die Arbeitsmaschine (2) umfassend eine Fahrerkabine (1) mit
- einer sich zwischen zwei Tragsäulen (3, 4) erstreckenden Frontscheibe (5),
- einem Fahrersitz (6),
- mindestens einem Visualisierungssystem (7) zur Visualisierung von Informationen für einen Maschinenführer,
- einem Überwachungssystem (8) zur Erfassung von Informationen betreffend eine physische Verfassung des Maschinenführers sowie
- einer Steuereinrichtung (9), die mit dem Visualisierungssystem (7) und dem Überwachungssystem (8) jeweils in Daten übertragender Weise verbunden ist,
**dadurch gekennzeichnet, dass**
die Steuereinrichtung (9) in Abhängigkeit einer erkannten physischen Verfassung des Maschinenführers das Visualisierungssystem (7) ansteuert, sodass das Visualisierungssystem (7) mindestens eine Handlungsempfehlung visualisiert, wobei die Handlungsempfehlung vorgesehen und eingerichtet ist, die Aufmerksamkeit des Maschinenführers und kurzfristig oder langfristig die Produktivität des Maschinenführers zu erhöhen und wobei das Visualisierungssystem (7) eine Scheibenanzeige (14) zur Visualisierung der die Handlungsempfehlung umfassenden Information umfasst und die Information in einem Blickfeld des Maschinenführers visualisiert.

## Claims

1. A driver's cab (1) for an agricultural working machine (2), comprising
- a front panel (5) extending between two support columns (3, 4),
- a driver's seat (6),
- at least one visualisation system (7) for visualising information for a machine operator,
- a monitoring system (8) for detecting information concerning a physical state of the machine operator, as well as
- a control device (9) which is connected to the visualisation system (7) and the monitoring system (8) respectively in a data-transmitting manner,
**characterized in that**
the control device (9) is configured to process information detected by the monitoring system (8) and to control the visualisation system (7) as a function of the information, so that at least one recommended action is visualised by means of the visualisation system (7), wherein the recommended action is provided and configured to increase the attentiveness of the machine operator and to increase the productivity of the machine operator in the short term or in the long term, and wherein the visualisation system (7) comprises a panel display (14) for visualising the information comprising the recommended action and the information is visualised in a field of view of the machine operator.

2. The driver's cab according to one of the preceding claims, the monitoring system (8) comprising a portable unit which the machine operator can carry, in particular in the form of a fitness tracker which can be worn on the wrist, by means of which at least a heart rate and/or a blood pressure of the machine operator can be detected.

3. The driver's cab (1) according to one of the preceding claims, **characterized in that** the monitoring system (8) includes an observation of the eyes, with which information concerning at least one parameter of the eye, in particular a pupil size of a particular eye, and/or a viewing direction of the machine operator, can be detected.

4. The driver's cab (1) according to claim 3, **characterized in that** the control device (9) is configured to control the visualisation system (7) as a function of information concerning the viewing direction of the machine operator detected by the monitoring system (8), wherein the visualisation system (7) is configured to track the visualisation of at least one item of information with the viewing direction of the machine operator.

5. The driver's cab (1) according to one of the preceding claims, **characterized by** a sensor device (26) for detecting information concerning a position of the driver's seat (6), wherein the control device (9) is connected to the sensor device (26) in a data-transmitting manner and is also configured to process information detected by the sensor device (26) and to control the visualisation system (7) as a function of the information, so that information visualised by means of the visualisation system (7) is tracked with a position of the driver's seat (6).

6. The driver's cab (1) according to one of the preceding claims, **characterized by** at least one audio system (17) for the acoustic reproduction of information for the machine operator, wherein the audio system (17) is connected to the control device (9) in a data-transmitting manner.

7. The driver's cab (1) according to claim 6, **characterized in that** the control device (9) is configured to control the audio system (17) as a function of the information detected by the monitoring system (8), so that at least one recommended action is reproduced acoustically by means of the audio system (17).

8. The driver's cab (1) according to one of the preceding claims, **characterized in that** the control device (9) can be operated in different working modes, wherein the control device (9) is configured to control the visualisation system (7) as a function of a working mode which is active at any one time so as to prioritise the visualisation of information which is relevant to the active working mode.

9. The driver's cab (1) according to claim 8, **characterized in that** at least one working mode is formed by a machine mode and/or an office mode and/or a relaxation mode, wherein a focus is on the operation of the working machine (2) in the working mode, on the execution of office tasks in the office mode and on the relaxation of the machine operator in the relaxation mode.

10. The driver's cab (1) according to claim 8 or claim 9, **characterized in that** the control device (9) is configured to control the visualisation system (7) as a function of a selected working mode, so that an item of information visualised by the visualisation structure is changed, wherein preferably, at least one item of information is enlarged and/or at least one item of information is hidden and/or at least one item of information is visualised in a field of view of the machine operator.

11. The driver's cab (1) according to one of claims 8 to 10, **characterized in that** the control device (9) is configured to control the visualisation system (7) in a manner such that information visualised by the visualisation structure can be changed, wherein at least one item of information can be shown and/or another item of information can be hidden.

12. The driver's cab (1) according to one of the preceding claims, **characterized in that** the visualisation system (7) comprises at least one panel display (14) cooperating with the front panel (5) and/or a side panel (13), in particular in the form of a display film laminated into the front panel (5) or the side panel (13), by means of which the information can be visualised.

13. The driver's cab (1) according to one of the preceding claims, **characterized in that** the visualisation system (7) comprises at least one display (15, 16), in particular in the form of a LC display, which is disposed on a support column (3, 4), preferably integrated into a support column.

14. The driver's cab (1) according to one of the preceding claims, **characterized by** at least one input unit which is operatively connected to the control unit (9), preferably in the form of at least one operating element (12) disposed on an armrest (11) of the driver's seat (6), by means of which the machine operator can carry out inputting.

15. The driver's cab (1) according to one of the preceding claims, **characterized in that** a drive system (18) is associated with the driver's seat (6), by means of which the driver's seat (6) can be adjusted in a motorised manner, wherein the drive system (18) is connected to the control device (9) in a data-transmitting manner, so that the driver's seat (6) can be adjusted by controlling the drive system (18).

16. The driver's cab (1) according to claims 9 and 15, **characterized in that** the control device (9) is configured to control the drive system (18) as a function of an active working mode.

17. An agricultural working machine (2),
**characterized by**
a driver's cab (1) according to one of the preceding claims.

18. A method for operating an agricultural working machine (2), the working machine (2) comprising a driver's cab (1) with
- a front panel (5) extending between two support columns (3, 4),
- a driver's seat (6),
- at least one visualisation system (7) for visualising information for a machine operator,
- a monitoring system (8) for detecting information concerning a physical state of the machine operator, as well as
- a control device (9) which is connected to the visualisation system (7) and the monitoring system (8) respectively in a data-transmitting manner,
**characterized in that**
the control device (9) controls the visualisation system (7) as a function of a detected physical state of the machine operator, so that the visualisation system (7) visualises at least one recommended action, wherein the recommended action is provided and configured to increase the attentiveness of the machine operator and to increase the productivity of the machine operator in the short term or in the long term, and wherein the visualisation system (7) comprises a panel display (14) for visualising the information comprising the recommended action and the information is visualised in a field of view of the machine operator.

## Revendications

1. Cabine de conduite (1) pour une machine de travail agricole (2), comprenant
- un pare-brise (5) s'étendant entre deux montants de support (3, 4),
- un siège de conducteur (6),
- au moins un système de visualisation (7) destiné à l'affichage d'informations pour un conducteur de machine,
- un système de surveillance (8) destiné à recueillir des informations concernant une condition physique du conducteur de machine, et
- un dispositif de commande (9) qui est relié au système de visualisation (7) et au système de surveillance (8), respectivement de manière à transmettre des données,
**caractérisée en ce que**
le dispositif de commande (9) est conçu pour traiter des informations recueillies par le système de surveillance (8) et pour activer le système de visualisation (7) en fonction des informations, de manière à ce qu'au moins une recommandation d'action soit affichée à l'aide du système de visualisation (7), la recommandation d'action étant prévue et conçue pour augmenter l'attention du conducteur de machine et accroître la productivité du conducteur de machine pendant une courte durée ou une longue durée, et le système de visualisation (7) comprenant un affichage sur vitre (14) destiné à visualiser les informations comprenant la recommandation d'action, et l'information étant affichée dans un champ de vision du conducteur de machine.

2. Cabine de conduite selon une des revendications précédentes le système de surveillance (8) comprend une unité portable que le conducteur de machine peut porter, notamment sous la forme d'un appareil de suivi de condition physique pouvant être porté au poignet, qui permet de mesurer au moins une fréquence cardiaque et/ou une tension artérielle du conducteur de machine.

3. Cabine de conduite (1) selon une des revendications précédentes, **caractérisée en ce que** le système de surveillance (8) inclut une observation des yeux qui permet de recueillir des informations concernant au moins un paramètre des yeux, notamment une dimension de la pupille d'un œil respectif et/ou une direction du regard du conducteur de machine.

4. Cabine de conduite (1) selon la revendication 3, **caractérisée en ce que** le dispositif de commande (9) est conçu pour activer le système de visualisation (7) en fonction d'informations recueillies par le système de surveillance (8) concernant la direction du regard du conducteur de machine, le système de visualisation (7) étant conçu pour faire en sorte que la visualisation d'au moins une information suive la direction du regard du conducteur de machine.

5. Cabine de conduite (1) selon une des revendications précédentes, **caractérisée en ce qu'**elle comporte un dispositif à capteur (26) destiné à recueillir des informations concernant une position du siège de conducteur (6), le dispositif de commande (9) étant relié au dispositif à capteur (26) de manière à transmettre des données, et étant conçu pour traiter des informations recueillies par le dispositif à capteur (26) et pour activer le système de visualisation (7) en fonction des informations recueillies, pour faire en sorte que des informations affichées à l'aide du système de visualisation (7) suivent une position du siège de conducteur (6).

6. Cabine de conduite (1) selon une des revendications précédentes, **caractérisée en ce qu'**elle comporte au moins un système audio (17) destiné à la reproduction acoustique d'informations pour le conducteur de machine, le système audio (17) étant relié au dispositif de commande (9) de manière à transmettre des données.

7. Cabine de conduite (1) selon la revendication 6, **caractérisée en ce que** le dispositif de commande (9) est conçu pour activer le système audio (17) en fonction des informations recueillies par le système de surveillance (8), de manière à ce qu'au moins une recommandation d'action soit reproduite sous une forme acoustique à l'aide du système audio (17).

8. Cabine de conduite (1) selon une des revendications précédentes, **caractérisée en ce que** le dispositif de commande (9) peut être utilisé dans différents modes de travail, le dispositif de commande (9) étant conçu pour activer le système de visualisation (7) en fonction d'un mode de travail respectivement actif, de manière à ce que des informations pertinentes pour le mode de travail actif soient visualisées de manière prioritaire.

9. Cabine de conduite (1) selon la revendication 8, **caractérisée en ce qu'**au moins un mode de travail est constitué d'un mode machine et/ou d'un mode bureau et/ou d'un mode détente, sachant qu'en mode de travail l'accent est mis sur la commande de la machine de travail (2), en mode bureau l'accent est mis sur la réalisation de tâches de bureau, et en mode détente l'accent est mis sur la détente du conducteur de machine.

10. Cabine de conduite (1) selon la revendication 8 ou 9, **caractérisée en ce que** le dispositif de commande (9) est conçu pour activer le système de visualisation (7) en fonction d'un mode de travail sélectionné, de manière à ce qu'une structure de visualisation d'informations affichées soit modifiée, de préférence au moins une information étant agrandie et/ou au moins une information étant supprimée et/ou au moins une information étant visualisée dans un champ de vision du conducteur de machine.

11. Cabine de conduite (1) selon une des revendications 8 à 10, **caractérisée en ce que** le dispositif de commande (9) est conçu pour activer le système de visualisation (7), de manière à ce qu'une structure de visualisation d'informations affichées puisse être modifiée, au moins une information pouvant être insérée et/ou au moins une autre information pouvant être supprimée.

12. Cabine de conduite (1) selon une des revendications précédentes, **caractérisée en ce que** le système de visualisation (7) comprend au moins un affichage sur vitre (14) coopérant avec le pare-brise (5) et/ou une vitre latérale (13), notamment sous la forme d'un film d'affichage intégré par stratification dans le pare-brise (5) ou la vitre latérale (13), à l'aide duquel des informations peuvent être visualisées.

13. Cabine de conduite (1) selon une des revendications précédentes, **caractérisée en ce que** le système de visualisation (7) comprend au moins écran (15, 16), notamment sous la forme d'un écran LCD, qui est placé sur un montant de support (3, 4) et est de préférence intégré dans un montant de support.

14. Cabine de conduite (1) selon une des revendications précédentes, **caractérisée en ce qu'**elle comprend au moins une unité de saisie (10) qui est en liaison fonctionnelle avec le dispositif de commande (9), de préférence sous la forme d'au moins un élément de commande (12) qui est disposé sur un accoudoir (11) du siège de conducteur (6) et permet au conducteur de machine de procéder à des saisies.

15. Cabine de conduite (1) selon une des revendications précédentes, **caractérisée en ce qu'**est associé au siège de conducteur (6), un système d'entraînement (18) qui permet de régler le siège (6) par moteur, le système d'entraînement (18) étant relié au dispositif de commande (9) de manière à transmettre des données, de manière à ce que le siège de conducteur (6) puisse être réglé par activation du système d'entraînement (18).

16. Cabine de conduite (1) selon les revendications 9 et 15, **caractérisée en ce que** le dispositif de commande (9) est conçu pour activer le système d'entraînement (18) en fonction d'un mode de travail actif.

17. Machine de travail agricole (2),
**caractérisée en ce qu'**elle comprend une cabine de conduite (1) selon une des revendications précédentes.

18. Procédé pour faire fonctionner une machine de travail agricole (2), la machine de travail agricole (2) étant dotée d'une cabine de conduite (1) comprenant
- un pare-brise (5) s'étendant entre deux montants de support (3, 4),
- un siège de conducteur (6),
- au moins un système de visualisation (7) destiné à la visualisation d'informations pour un conducteur de machine,
- un système de surveillance (8) destiné à recueillir des informations concernant une condition physique du conducteur de machine, et
- un dispositif de commande (9) qui est relié au système de visualisation (7) et au système de surveillance (8), respectivement de manière à transmettre des données,
**caractérisé en ce que**
le dispositif de commande (9) active le système de visualisation (7) en fonction d'une condition physique détectée du conducteur de machine, de manière à ce que le système de visualisation (7) affiche au moins une recommandation d'action, la recommandation d'action étant prévue et conçue pour augmenter l'attention du conducteur de machine et accroître la productivité du conducteur de machine pendant une courte durée ou une longue durée, et le système de visualisation (7) comprenant un affichage sur vitre (14), destiné à visualiser l'information comportant la recommandation d'action, et visualisant l'information dans un champ de vision du conducteur de machine.
